(19)

**Europäisches Patentamt**

**European Patent Office**

**Office européen des brevets**

(11)  **EP 4 014 935 B1**

(12)  **EUROPEAN PATENT SPECIFICATION**

(45) Date of publication and mention of the grant of the patent:
**27.11.2024  Bulletin 2024/48**

(21) Application number: **20215636.0**

(22) Date of filing: **18.12.2020**

(51) International Patent Classification (IPC):
***A61F 13/15*** *(2006.01)*      ***A61F 13/20*** *(2006.01)*

(52) Cooperative Patent Classification (CPC):
**A61F 13/15699; A61F 13/2082**

(54) **IMPROVED METHOD FOR MANUFACTURING TAMPON**

VERBESSERTES VERFAHREN ZUR HERSTELLUNG EINES TAMPONS

PROCÉDÉ AMÉLIORÉ DE FABRICATION D'UN TAMPON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**22.06.2022  Bulletin 2022/25**

(73) Proprietors:
• **Ontex BV**
**9255 Buggenhout (BE)**
• **Ontex Group NV**
**9320 Erembodegem (BE)**

(72) Inventors:
• **Heege, Thomas**
**56761 Düngenheim (DE)**
• **ADAMS, Christian**
**56729 Kreuznick (DE)**

(74) Representative: **Saurat, Thibault**
**Ontex BV**
**Legal Department**
**Korte Keppestraat 21**
**9320 Erembodegem (BE)**

(56) References cited:
**EP-A1- 3 184 092      EP-A1- 3 412 264**
**US-A1- 2014 202 123**

EP 4 014 935 B1

**Description**

**TECHNICAL FIELD**

The invention pertains to the technical field of manufacturing tampon blanks, tampons, specifically digital tampon, and intermediate products thereof. The invention relates to an improved method for producing tampon blanks, and an apparatus for doing so, as well as the product manufactured by said method.

**BACKGROUND**

Tampons are well known in the art and are used for feminine hygiene. Also many tampon manufacturing methods and apparatuses have been disclosed in the prior art. Generally, a distinction is made between folded and rolled tampons. The former have improved absorbent characteristics, but possess less strength and are commonly used with an applicator to reduce the chance of tears and other damage to the tampon before insertion. Rolled tampons are slightly less absorbent, but more sturdy and can be applied digitally, as opposed to the folded tampons. Furthermore, measures can be taken to increase the absorbency of the rolled tampons. The invention will focus on uses concerning rolled tampons and intermediate products thereof.

Rolled tampons comprise a rolled absorbent fiber sheet and a withdrawal string for removal. Rolled tampons are manufactured by rolling multiple-layered sheets. The multiple-layered sheets comprise a layer of absorbent material of a certain length, upon which a strip of web material is bonded which has only a fraction of the length of the layer of the absorbent material, thus creating the multiple-layered sheets. A further description of this product will be provided later in this document.

It is desired to attain a maximal production speed, but one recurring problem is that the machines are often subject to mechanical failure especially during the bonding of the thermoplastic film onto the absorbent material. For example EP 1 189 566 describes a process with ironing plates meant to heat up and melt a thermoplastic film onto the absorbent material. EP 3 165 471 also describes a process where a heating element is pressed against a wound tampon blank to seal the free-end of the strip onto the absorbent material. The melted thermoplastic film is thereby bonded with the absorbent material. The issue in these processes is that a portion of the melted film sticks onto the ironing plates creating a layer of molten film which decreases the heating yield in time thus resulting in a worst bonding of the film onto the absorbent material.

Document EP3184092 describes a method for intermittently providing strips of a continuous first web material onto a continuous second web material for manufacturing tampon blanks however the efficiency of this method needs to be improved.

The invention thereto aims to provide a method and apparatus which ensures an efficient and reliable bonding of the film material onto the absorbent material.

**SUMMARY OF THE INVENTION**

The present invention provides a method for manufacturing a tampon, comprising the steps of:

- supplying a continuous first web material;
- supplying a continuous second web material;
- cutting strips of the continuous second web material, whereby the strips are cut transversally, preferably perpendicularly, with respect to a longitudinal axis of the continuous second web material and equidistantly repeated over the longitudinal axis of the continuous second web material;
- bonding a first-end of the strip of second web material to the continuous first web material, thereby forming a partially laminated structure at a bonding station;
- conveying said partially laminated structure to a winding station;
- clamping the partially laminated structure by rotatable clamping means and rolling-up the clamped partially laminated structure, thereby separating the partially laminated structure into strips along breaking points;
- winding the partially laminated structure strip so that the strip of second web material forms the outer layer around the first web material;
- transferring the wound partially laminated structure strip to a sealing station; and
- sealing the second-end of the strip of second web material to the first web material and/or second web material.

According to the invention, the sealing step comprises ultrasonic bonding, wherein the bonding step concurrently comprises an embossing step where the bonding station comprise an embossment roller that has spaced apart protrusions, the partially laminated structure being arranged in a way that the first web material comes into contact with the embossment roller.

The method according to the present invention allows an adequate melting of the second web material onto the first web material or vice versa. Indeed, vibrations can be induced at a distance, therefore it is possible to melt the second web material at a distance therefore suppressing any stagnant layer of molten web on the heating element. This results in an improved sealing of bonding of the film onto the absorbent material.

According to an embodiment, the sealing station comprises at least one sealing sonotrode inducing vibrations at a frequency between 10 kHz and 50 kHz, preferably between 20 kHz and 35 kHz.

According to an embodiment, the sealing station comprises three or five sealing sonotrodes inducing vibrations at a frequency between 10 kHz and 50 kHz, preferably between 20 kHz and 35 kHz.

According to an embodiment, the at least one sealing sonotrode is mounted mobile within an arcuate railing and is configured to move at an constant distance relative to the rotatable clamping means.

According to an embodiment, the at least one sealing sonotrode is mounted mobile within an at least partially linear rail and is configured to move closer or remoter to the rotatable clamping means.

According to an embodiment, the continuous second web material is supplied at a second speed, whereby said second speed is greater than zero, preferably whereby said second speed is constant; the continuous first web material is supplied at a first speed, which is higher than the second speed, preferably whereby said first speed is constant, the method comprising a step of accelerating the strips from the second speed to the first speed; wherein the step of accelerating the strips from the second speed to the first speed is conducted essentially concurrently with the step of bonding a first-end of the strip of second web material to the continuous first web material, thereby forming a partially laminated structure.

According to an embodiment, the method further comprises a step of applying predetermined breaking points on the continuous first web material, whereby the predetermined breaking points are applied transversally with respect to a longitudinal axis of the continuous first web material and equidistantly repeated over the longitudinal axis of the continuous first web material, and whereby said step of applying predetermined breaking points is executed prior to the bonding step.

The invention also pertains to an apparatus for manufacturing a tampon, comprising:

- a first conveying system comprising:

  ◦ one or more conveying means, preferably comprising one or more rollers, for conveying a first web material to a bonding station;

- a second conveying system comprising:

  ◦ one or more conveying means, preferably comprising one or more rollers, for conveying the second web material to the bonding station;
  ◦ a separation station, preferably comprising a cutting roller and a counterpressure roller, for cutting strips of second web material, preferably configured for cutting said strips equidistantly and transversally, more preferably perpendicularly, with respect to a conveying direction of the second web material;

- a bonding station, for bonding a first-end of the strip of second web material to the first web material, thereby forming a partially laminated structure;
- a winding station comprising rotatable clamping means, for clamping and winding the partially laminated structure;
- a sealing station, for sealing the second-end of the strip of second web material to the first web material and/or second web material.

According to the invention, the sealing station comprises an ultrasonic welding device inducing vibrations, wherein the bonding station comprises an embossment roller comprising a smooth surface and a embossed surface, the embossed surface comprising an embossment pattern with at least one row of protrusions, said protrusions being arranged in the same orientation for each row and being spaced apart from one another.

According to an embodiment, the ultrasonic welding device is at least one sealing sonotrode inducing vibrations at a frequency between 10 kHz and 50 kHz, preferably between 20 kHz and 35 kHz.

According to an embodiment, the apparatus further comprises a weakening station, said weakening station preferably comprising a weakening roller, more preferably a perforation roller, and a counterpressure roller, for applying predetermined breaking points on the first web material or on the partially laminated structure, whereby the predetermined breaking points are applied transversally with respect to a longitudinal axis of the continuous first web material and equidistantly repeated over the longitudinal axis of the continuous first web material.

According to an embodiment, the apparatus further comprise conveying means and preferably guiding means arranged between the bonding station and the winding station in order to convey the partially laminated structure from the bonding station to the winding station, said conveying means comprising at least two, preferably four rollers arranged two by two, the partially laminated structure passing through each pair or roller.

According to an embodiment, the sealing station comprises a reciprocating mechanism enabling the movement of the at least one sealing sonotrode closer or remoter to the rotatable clamping means.

According to an embodiment, the sealing station comprises a actuating mechanism enabling the movement of the sealing sonotrode at a constant length relative to the rotatable clamping means, i.e. the sealing sonotrode and the rotatable clamping means are separated by a gap that has a constant height.

According to an embodiment, the bonding station comprises an ultrasonic welding device to bond the first-end of the second web material to the first web material, the ultrasonic welding device comprising a bonding sonotrode inducing vibrations at a frequency between 10 kHz and 50 kHz, preferably between 20 kHz and 35 kHz.

All of these embodiments mentioned above can be taken individually or in combination.

Further embodiments are described below and in the claims.

## DESCRIPTION OF FIGURES

 FIG. **1** illustrates schematically from a side view a module, or a part, of the process to manufacture an absorbent article in accordance with an embodiment of the present invention.
 FIG. **2** illustrates a close-up of the module in Figure 1, namely the cutting of the second web material into strips.
 FIG. **3** illustrates the embossment roller according to an aspect of the invention.
 FIG. **4** illustrates a close-up of the embossment roller according to an aspect of the invention.
 FIG. **5** illustrates the embossment roller from a top view according to an embodiment of the invention
 FIG. **6** schematically illustrates a close-up of the module in Figure 1, namely the bonding station.
 FIG. **7** schematically illustrates the laminated structure resulting from the bonding step from a side view
 FIG. **8** schematically illustrates the laminated structure once wound from a side view
 FIG. **9** illustrates schematically from a side view another module, or a part, of the process to manufacture an absorbent article in accordance with an embodiment of the present invention.
 FIG. **10** illustrates a close-up of the module in Figure 9, namely the sealing station.
 FIG. **11** illustrates the tampon resulting from the sealing station.

## DETAILED DESCRIPTION OF THE INVENTION

The present invention concerns an improved method for producing a tampon, and an apparatus for doing so, as well as the product manufactured by said method.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The term "nonwoven web material" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The term "thermoplastic" is meant to describe a material that softens when exposed to heat and which substantially returns to its original condition when cooled to room temperature.

The term "rayon" refers to a manufactured regenerated cellulose fiber, made from purified cellulose. It has a smooth, soft surface, and is therefore very suitable to be used in a tampon.

The term "concurrently" or "essentially concurrently" refers to the simultaneous or overlapping occurrence or execution of two or more events or steps.

The term "speed", both the first speed as the second speed, refers to the speed at which the first or second web material moves. This can for instance be the tangential speed at the surface of conveying rollers (and/or possibly the separation roller and/or the weakening roller).

The term "first web material" can also refer to already cut strips of the continuous web material. This is to be noted as the cutting of the strips occurs very fast and sudden. The first web material may be continuous at the start of a process, such as being conveyed (or a first section of the first web material being conveyed), but can be cut during the process. As such, the term "first web material" covers both the continuous first web material as the strips of the first web material.

The term "predetermined breaking points" refers to areas of weakened tensile strength, which are applied along a line, in this case usually in a line transversal with respect to the longitudinal axis of the web material. This creates "breaking lines", which allows the web material to be separated more easily into strips or sections, for instance by tearing or cutting, while still allowing the web material to be conveyed at high speeds without tearing at inopportune times. These predetermined breaking points can be applied by several different processes, for instance by applying perforations through small cuts, or by weakening certain zones by scraping off some of the material, thus weakening it. In other cases the weakened zones can be achieved by applying heat and/or acid to reduce the tensile strength. Note that combinations of the aforementioned methods are also possible, as well as others which have not been mentioned but are considered to be common knowledge in the field.

The recitation of numerical ranges by endpoints includes all numbers and fractions subsumed within that range, as well as the recited endpoints.

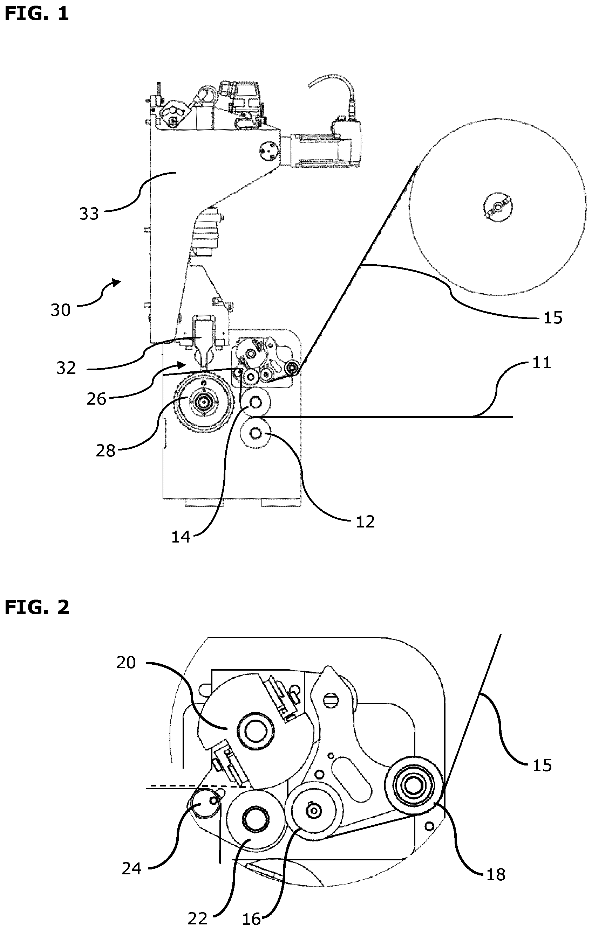

FIG. 1 illustrates schematically from a side view a module, or a part, of the apparatus and process to manufacture tampons, in accordance with an embodiment of the present invention. Specifically, FIG. 1 shows namely the bonding of a first web material 11 with a second web material 15. In FIG. 1, a possible configuration is shown of an apparatus for manufacturing tampon blanks. However, it is to be noted that not all of these elements are strictly necessary and/or could be replaced by other elements with similar functions in order to accomplish the objective of the invention, and that the invention is therefore not limited to this embodiment.

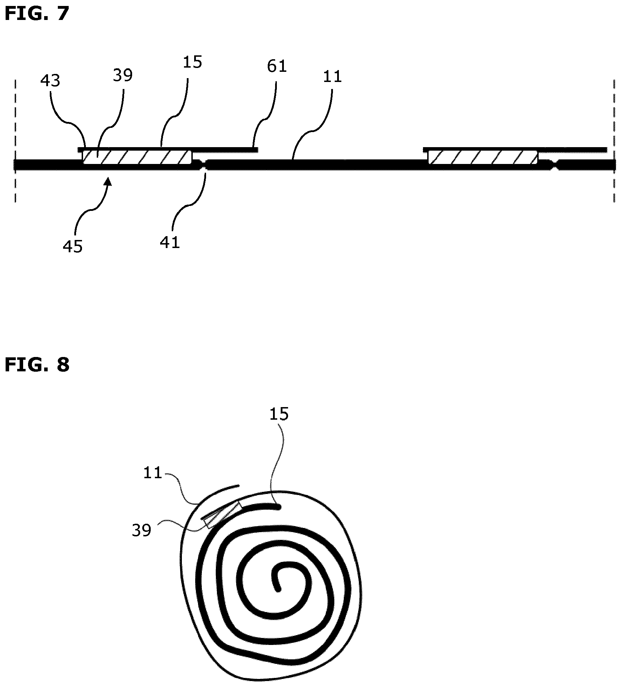

In the apparatus, a first web material 11 is provided continuously at a first speed to a weakening station 12,14. In this embodiment, the weakening station 12,14 comprises a weakening roller 12 and a counterpressure roller 14. The weakening roller in this case is a perforation roller 12 and comprises one or more (two, three, four or more) blades that apply a perforation line on the first web material 11 and the counterpressure roller 14 acts as an anvil. The weakening station 12,14 can also scrape off material in zones of the first web material 11, or treat the first web material 11 with acid or heat to create predetermined breaking points 41 (as seen in FIG. 7), or even employ a combination of more than one of said methods. In the preferred embodiment, the weakening station 12,14 is a perforation station and the perforation roller 12 comprises blades to form a zigzag pattern along the perforation line. The distance between subsequent perforation lines can be adapted by using a different perforation roller 12 with a different number of blades and/or a different diameter and/or different rotational speeds. Note however that the weakening station 12,14, or parts thereof, also acts as a conveying system for the first web material 11. After being perforated, the first web material 11 is conveyed over the counterpressure roller 14 of the weakening station 12,14 to the bonding station 26 that will be described afterwards. As illustrated in FIG.1, the weakening roller 12 rotates counter-clockwise whereas the counterpressure roller 14 rotates in the opposite direction, i.e. clockwise.

The invention is not limited to this specific embodiment and the rollers can be arranged differently. For example, in FIG. 1 the weakening roller 12 is arranged vertically under the counterpressure roller 14. This arrangement can be inverted where the counterpressure roller 14 can be arranged under the weakening roller 12.

According to another embodiment, the first web material 11 may be conveyed toward the bonding station 26 without passing through a weakening station. There can only be one roller here the one reference as 14, or two rollers 12,14 each comprising a smooth outer surface, and acting as pulling rollers conveying the first web 11 towards the bonding station 26. A weakening station can be implemented downstream of the bonding station 26.

In the apparatus, a second web material 15 is provided continuously at a second speed to a separation station 20,22 by a first conveying system 16,18. The separation station 20,22 is illustrated in FIG 2 which corresponds to a close-up of FIG. 1. In this embodiment, the first conveying system 16, comprises a pulling roller 16 for pulling/unwinding the second web material 15 from a web roll or other sources, and conveying it further along to the separation station. The first conveying system 16,18 can further comprise a guiding roller 18 for correctly guiding the second web material 15 to the pulling roller 16. The guiding roller 18 possibly comprises a guiding notch for centering the second web material 15 in case of deviation of the path of the second web material 15 while being pulled from the web roll or other sources. Subsequently, the second web material 15 is conveyed through the separation station 20,22, where a separation roller 20 cuts the second web material 15 into strips in concert with a counterpressure roller 22 acting as an anvil which conveys the second web material 15 and the cut strips and provides a base on which the second web material 15 can be cut by the separation roller 20. The separation roller 20 in this example comprises two blades placed at opposite sides of the separation roller 20, however other versions can be used, having one, three, four, five or more blades. Preferably, the separation roller 20 and the counterpressure roller 22 rotate at equal (or at least similar) tangential surface speeds in order to provide a clean cut without dragging the second web material 15. In order to change the length of the strips cut by the separation roller 20, a separation roller 20 with a different diameter and/or a different amount of blades and/or a different rotational speed can be used. When the second web material 15 has passed the separation station 20,22, not necessarily as a cut strip yet, the second web material 15 is conveyed further by a combining roller 24 towards the bonding station 26. The combining roller 24 is adapted to push the first web material 11 and the second web material 15 further along to the bonding station 26 where the strips of second web material 15 can be bonded onto the first web material 11. The combining roller 24 corresponds to the point in the method and apparatus where the first web material 11 and the second web material 15 are combined but not bonded yet.

The invention is not limited to this specific embodiment and the rollers 16,18,20,22,24 can be arranged differently. For example, in FIG. 2 the second web material 15 passes under the guiding roller 18, between the counterpressure roller 22 and the pulling roller 16, and then between the counterpressure roller 22 and the separation roller 20. This arrangement can be modified for example by taking out the guiding roller 18, the pulling roller 16 acting as a pulling and guiding roller or by arranging the pulling roller 16 at a distance from the separation station 20,22. As illustrated in FIG 2., the guiding roller 18, the pulling roller 16 and the separation roller 20 all rotate in the same direction, here clockwise, whereas the counterpressure roller 22 rotates in the opposite direction hence counter clockwise. Of course, each can be inverted as needed, for example the second web material 15 can be placed vertically under the guiding roller 18, thus going over the guiding roller 18, the guiding roller 18 would then rotate counter-clockwise.

The apparatus can further comprise an extension for example in the form of a ramp to collect the second web material 15 from the counterpressure roller 22. Furthermore, air blowing means can be implemented to provide constant air streams to press the second web material 15 against said extension or against the first web material 11, thus reducing the risk of the second web material 15 curling up.

The combined first web material 11 and second web material 15 are conveyed by the combining roller 24 towards the bonding station 26. The bonding station 26 comprises an embossed roller 28 and a ultrasonic welding device 30 as illustrated in FIG. 1. The ultrasonic welding device 30 comprises a vibration generator 33, for example and not limited to, a magnetostrictive or piezoelectric transducer, said generator 33 is attached to a tapering rod or probe usually made out of metal, such as and not limited to titanium, aluminum or steel, that will be referenced herein as the bonding sonotrode 32. The bonding sonotrode 32 is submitted to the vibrations generated by the generator 33 and said bonding sonotrode 32 then applies this vibrational energy to the combined first and second web material 11,15. The vibrational energy causes the partial melting of the second web material 15 so as to bond the first and second web material 11,15 in these melting points.

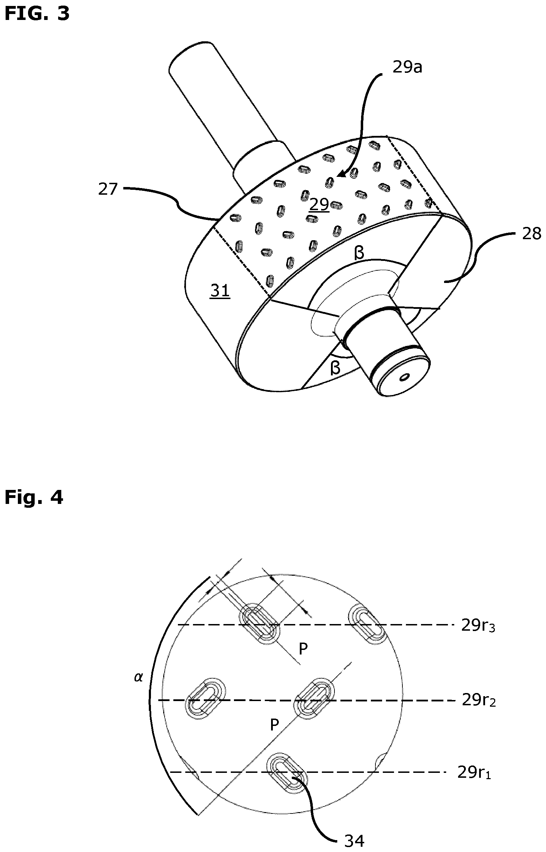

The melting points, which can be considered as bonding regions 39 (see FIG. 7), are defined by the embossment pattern 29a of the embossment roller 28. The embossment roller 28 as illustrated on FIG. 3, comprises at least one embossed surface 29 comprising an embossment pattern 29a and at least one smooth surface 31 lacking any embossment. Preferably, the embossment roller 28 comprises two identical embossment patterns 29a, or two embossed surface 29, arranged on opposite sides of the roller 28. The embossment pattern 29a corresponds to protrusions 34, in other words protuberances protruding in a radial direction from the smooth surface of the embossment roller 28, said protrusions 34 being spaced apart from one another.

The protrusions 34 correspond here to truncated elliptical cones that decrease in surface from the base, corresponding to the surface of the embossment roller 28, up to its apex. As illustrated in FIG. 3, each protrusion 34 is identical to the other and presents an oblong surface at its apex. Starting from the apex, each protrusion 34 widens in direction of the base. Each protrusion 34 presents at its apex an oblong surface with two opposite sides being larger than the two other opposite sides, in other words, the apex surface of each protrusion extends, or is elongated, sensibly in one direction of axis P as illustrated in FIG. 4.

According to one embodiment, as illustrated in FIG. 3, the protrusions 34 are arranged in rows. More precisely, the protrusions 34 are aligned in n rows, where n is natural number, or positive integer. In other words, the embossment pattern 29a can comprise 1, 2, 3, 4, 5, 6, 7, 8, 9, 10 or more rows. As illustrated in FIG. 3, the embossment pattern 29a comprises four rows $29r_m$. Each row $29r_m$ comprises m protrusions 34, where m is natural number, or positive integer. In other words, each row $29r_m$ can comprise 1, 2, 3, 4, 5, 6, 7, 8, 9, 10 or more protrusions 34. As illustrated in FIG. 3, the embossment pattern 29a comprises four rows $29r_m$, two rows comprise eight protrusions 34 and two rows comprise seven protrusions 34.

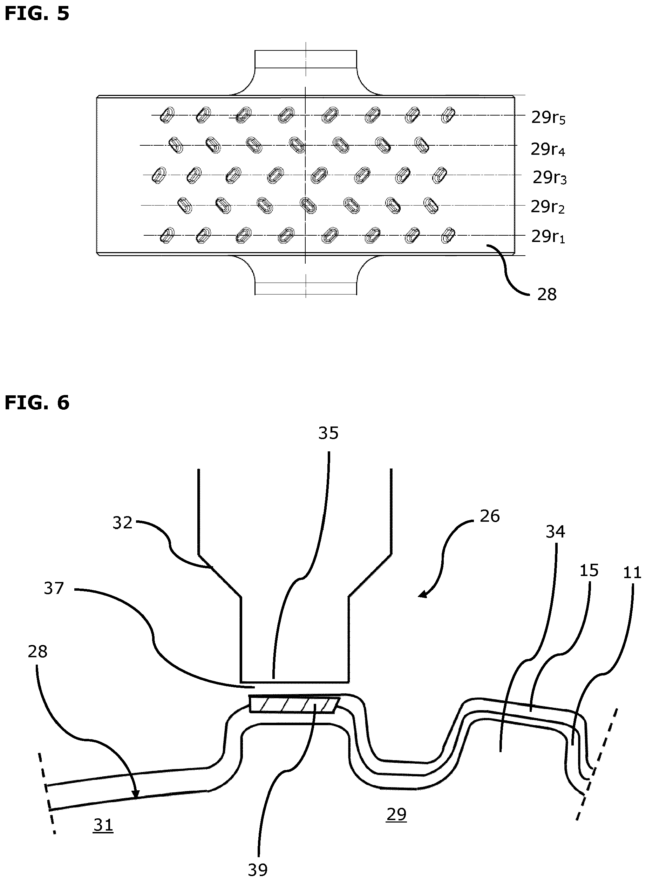

The invention is not limited to this specific embodiment, the embossment pattern 29a can comprise five rows $29r_m$ of protrusions 34 as illustrated in FIG. 5, and the rows of protrusions 34 can have the same number of protrusions 34 or a different number of protrusions 34 from one another.

The protrusions 34 can be arranged in a staggered configuration, where the rows $29r_m$ are aligned two by two with two adjacent rows being shifted by a gap or pitch. In other words, five adjacent protrusions 34 can define a quincunx pattern or matrix, especially when the shifting gap corresponds to half the length, or interval, separating two protrusions 24 as illustrated in FIG.4. In other words, the protrusions 34 of the first 29ri and are aligned with the protrusions of the third row $29r_3$ and the protrusions 34 of the second row 29rz and are aligned with the protrusions of the fourth row $29r_4$. The rows $29r_m$ can be arranged in an irregular staggered configuration, where the shifting gap is different from one row to another, as illustrated in FIG.4 or 5 where the first 29ri and third row $29r_3$ do not have the same shifting gap in regards to the second row 29rz. In other words, the protrusions 34 of the first row 29ri and are not aligned with the protrusions of the third row $29r_3$ and the protrusions 34 of the second row 29rz and can be aligned or not with the protrusions of the fourth row $29r_4$, the invention not being limited to a particular embodiment.

In a row $29r_m$, the protrusions are aligned and oriented in the same way, i.e. the axis P of each protrusions 34 in a row $29r_m$ are parallel to each other. In other words, for each row $29r_m$, the protrusions 34 are arranged in the same orientation, each protrusion being spaced apart by an interval, said interval being equal or different from one protrusion 34 to the other. According to an embodiment, the protrusion 34 from one row $29r_m$ to the next is rotated by an angle $\alpha$ comprised between 0° and 180°, preferably between 30 and 135°, more preferably between 45° and 120° and even more preferably at 90° as illustrated in FIG. 4. In the embodiment as illustrated in FIG. 4, the axis P of protrusions 34 that are in two rows $29r_m$ separated by another row $29r_m$ in between are parallel, in other words, the axis P of the protrusions 34 in the row 29ri is parallel to the axis P of the protrusions 34 in the row $29r_3$, the axis P of the protrusions 34 in the row 29rz being perpendicular to the axis P of the protrusions 34 in the row 29ri and row $29r_3$. According to another embodiment, the protrusions 34 all present the same orientation, *i.e.* all the axis P are parallel to one another.

The row $29r_m$ and/or protrusions 34 can be evenly distributed, as illustrated in Fig. 5, or irregularly distributed over the embossment pattern 29a.

The embossment pattern 29a, thus embossed surface 29, spans over a certain degree β of the embossment roller 28. Preferably, the embossment pattern 29a spans over 50° to 180° of the embossment roller 28, more preferably, the embossment pattern 29A spans over 80° to 140°, even more preferably, the embossment pattern 29a spans over 120° and 130°, for example 125°. In the embodiment where the embossment roller 28 comprises two embossment patterns 29a arranged on opposite side of the roller, said embossment patterns 29a, or embossed surfaces 29, preferably span over the same circumferential length, i.e. the two embossment patterns 29a spans over the same degree value of the embossment roller 28. This enables to have a laminated structure (that will be described after) that comprises a proper proportion of bonded and unbonded first web material 11 as illustrated in FIG. 7. According to an alternative embodiment, the embossment patterns 29a can span over different degree value of the embossment roller 28.

The embossment roller 28 comprises a succession of embossed surface 29 and smooth surface 31. The embossment roller comprises at least one embossed surface 29 and at least one smooth surface 31. Preferably, the embossment roller can comprise two embossed surfaces 29 and two smooth surfaces 31 where the embossed surfaces 29 are on opposite sides of the roller 28 and the smooth surfaces 31 are on opposite sides of the roller 28, each smooth surface 31 being arranged between two embossed surfaces 29 and each embossed surface 29 being arranged between two smooth surfaces 31. Of course the embossment roller 28 can comprise three, four, five or more embossed surfaces 29 and smooth surfaces 31. Preferably, there is the same number of smooth surfaces 31 and embossed surfaces 29 on the embossment roller 28. According to an alternative there can be more smooth surfaces 31 than the embossed surface or vice versa on the embossment roller 28.

In one embodiment, the embossment roller 28 comprises a first embossed surface 29 covering 125° of the embossment roller 28, a smooth surface 31 covering 55° of the embossment roller 28, another embossed surface 29 also covering 125° of the embossment roller 28 and another smooth surface 31 also covering 55° of the embossment roller 28. In this embodiment, the embossed surfaces 29 cover a bigger portion of the embossment roller 28 than the smooth surfaces 31 (250° to 110°). The ratio r between the portion covered by the embossed surfaces 29 and the portion covered by the smooth surface is superior or equal to 1, in other words the embossment roller 28 respects the following relation:

$$r\left(\frac{Embossed\ Portion\ (29)}{Smooth\ Portion\ (31)}\right) \geq 1.0$$

. Preferably the ratio is comprised between 1.2 and 4, more preferably, the ratio is comprised between 1.5 and 3.0, even more preferably, the ratio is comprised between 2.1 and 2.4. For example, the portion of the embossment roller 28 covered by all the embossed surfaces 29 can cover 2.3 times the portion of the embossment roller 28 covered by all the smooth surfaces 31. As explained above, the embossed portion of the embossment roller 28 corresponds to the bonding region 39 between the first and second web material 11,15. It is preferable to have a higher bonding ratio. Although it could be possible to have an embossment roller 28 without any smooth surface, i.e. the embossed pattern 29a spans over the entire circumference of the embossment roller 28, it is preferable to have a certain portion of smooth surface 31, meaning non-bonded portions, for the sealing step that will be explained hereunder.

The first web material 11 and the strip of second web material 15 are combined at the combining roller 24, the strips of second web material 15 laying on top of the first web material 11 forming a pre-bonded or combined structure. The combined structure is guided towards the bonding station 26. As illustrated in FIG. 1 and 6, the bonding station 26 comprises the embossment roller 28 and the ultrasonic welding device 30 which comprises an ultrasonic vibration generator 33 and the bonding sonotrode 32, or probe. The ultrasonic welding device 30 is mounted vertically above the embossment roller 28, leaving a small gap 37 between the outer surface of the embossment roller 28 and the bonding sonotrode 32. The first web material 11 comes in contact with the embossment roller 28 either at a smooth surface 31, at an embossed surface 29 or at their junction 27. The protrusions 34 of the embossed surface 29 cause a local elevation of the second and first web material 11,15 as illustrated in FIG 6. While the bonding sonotrode 32 is constantly expanding and contracting at a certain frequency, the first and second web materials 11,15 are conveyed through the gap 37 between the bonding sonotrode 32 and the embossment roller 28.

During the expansion phase, the bonding sonotrode 32 compresses the second web material 15 between the welding surface 35 of the bonding sonotrode 32 and first web material 11 as well as the embossment roller 28. This compression creates molecular compression and surface friction, causing the second web material 15 to selectively melt at the protrusions 34 of the embossment roller 28. More precisely, the compression and friction of the molecules create heat that melts selectively the second web material 15 at each protrusion 34 positioning. For this purpose, the second web material 15 comprises preferably, but not limited to, a nonwoven thermoplastic material, for example PolyEthylene (PE), Polyester (PET for example) or PolyPropylene (PP), or bicomponent fibres, for example and not limited to PE/Polyester. In a possible embodiment, the second web material 15 is a soft carded thermobonded nonwoven material with a smooth surface. The smooth surface aids in the application of the hygienic product. The second web material 15 is preferably hydrophilic, and can for instance be a 100% mix of polyethylene (PE) and polyester, preferably polyethylene terephthalate (PET). Further characteristics of the second web material of the proposed embodiment is an area density of about 12 g/m$^2$ (according to WSP 130.1), a tensile strength at maximal force in the machine direction (lengthwise) of about 16 N/5 cm, a tensile strength at maximal force in the cross machine direction (breadthwise) of about 2 N/5 cm, an elongation at maximal force in the machine direction of about 25%, an elongation at maximal force in the cross machine direction of about 30% (all according to WSP 110.4) and a liquid strike-through time (according to WSP 70.3) of about 2.5 s. These characteristics have been measured by WSP protocols.

According to one embodiment, the first web material 11 and the second web material are of different nature. More precisely, the first web material 11 comprises an absorbent material such as rayon, polyester or cotton fibers whereas the second web material 15 comprises a thermoplastic material as described above. The second web material 15 melts at lower temperature (100-150°) whereas the first web material 11 melts at higher temperature (around 200°), therefore the vibrations transmitted by the bonding sonotrode 32 will melt specifically the second web material 15. During the contraction phase, the bonding sonotrode 32 enables a bigger gap 37, allowing the web materials to run through the gap at high speeds without material jams. With the local melting of the second web material 15, said melting web material 15 penetrates into the voids present between the fibres of the first web material 11 thereby creating a bonding region 39 between the two web materials 11,15. In other words, the bonding station 26 enables the formation of an at least partially laminated structure 45 where the first-end 43 of the strip of second web material 15 is bonded onto the first web material 11.

The amount of energy brought into the second web material 15 depends upon the amplitude of the bonding sonotrode 32 and the force applied said material 15. While the amplitude remains constant, the bond strength can be regulated by adjusting the gap 37. Indeed, if the gap 37 is too high, less energy is applied to the second web material 15 resulting in weaker bonds whereas, if the gap 37 is too small, the bond will be stronger but there's a higher risk of jamming.

The ultrasonic welding device 30 can be calibrated to have an adequate gap 37, or the ultrasonic welding device 30 can comprise an actuator unit with a height adjustment system configured to change the operating position of the bonding sonotrode 32 via a toggle mechanism.

The partially laminated structure 45 resulting from the bonding station 26 is as illustrated in FIG. 7. It will be understood in the rest of the description that a partially laminated structure 45 corresponds to a partially bonded structure 45 or to a partially bonded first and second web material 11,15 without any limitation or restriction imposed to a term or its equivalent.

In this embodiment, the first web material 11 is provided with predetermined breaking points 41, preferably along a line transversal to its longitudinal axis, equidistantly along its longitudinal axis, and is intermittently provided with strips of the second web material 15, of a different length than between subsequent predetermined breaking points 41. In this embodiment, the strips of second web material 15 are shorter than the distance between the subsequent predetermined breaking points 41 of the first web material 11. A first-end 43 of strips of second web material 15 is partly bonded, passing through the bonding station 26 described hereabove, to the first web material 11. Preferably, the bonding region 39 is arranged so that a part of the strip of second web material 15 extends beyond said end of the first web material 11 defined by the predetermined breaking points 41 as illustrated in FIG. 7. Possible dimensions for the tampon are a length between subsequent predetermined breaking points 41 comprised between 220 and 280 mm, preferably between 240 and 260 mm, more preferably 248 mm, 249mm or 250 mm, for the first web material 11, and a length comprised between 100 and 140 mm, preferably between 120 and 130 mm, more preferably 124 mm or 125 mm for the strip 15 of second web material. The part of the strip of second web material 15 that is bonded 39 to the first web material 11 can for instance extend over a distance of 70 mm, up to 90 mm. The part of the strip of second web material 15 extending over the predetermined breaking point can extend over a distance of 30 mm. This configuration is possible by adjusting the ratio r (embossed surface/smooth surface) of the embossment roller 28. In a particular embodiment, the tampon has the following dimensions: a length of 248 mm for the first web material 11 between two breaking points 41, a length of 124 for the second web material 15 and a length of 84 mm for the bonded region 39, the length of unbonded second web material 15 is of 40 mm in this embodiment. The first web material 11 is preferably wider than the second web material 15. The width of the first web material 11 is comprised between 45 and 52 mm whereas the width of the second web material 15 is comprised between 35 and 45 mm.

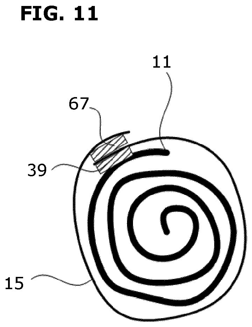

It is preferable for the production process that the first web material 11 is provided with predetermined breaking points 41, but not entirely separated, or too strongly weakened, in order for said second web material 15 to be pulled through the apparatus as a continuous stream. After the strips of the second web material 15 are bonded, it is possible to fully separate the first web material 11 at the predetermined breaking points 41, thus creating identical partially laminated structure 45, comprising both the first 11 and the second web material 15 bonded at the first-end 43 of the strip of second web material 15. This partially laminated structure 45 can subsequently be rolled, according to a winding process that is detailed hereunder, in a matter as illustrated in FIG. 8, with the first web material 11 on the interior of the tampon blank, and the second web material 15 covering the first web material 11 as an outer layer. After the tampon blank has been rolled, a sealing operation that is described hereunder can be executed to seal the outer layer of the second web material 15, the original strip, as can be seen in FIG. 11.

By carefully choosing the outer layer of second web material 15 and the first web material 11 itself, many advantageous effects are achieved, for instance it increases the stability of the tampon, thus reducing deformations and generating a more aesthetically pleasing appearance, which is instrumental in appealing to a customer and providing the assurance of quality of a hygienic and very intimate product. Furthermore, the application of the tampon is easier as the outer layer of the second web material 15 is smoother than the interior first web material 11. This way, application of the tampon is made easier, while still allowing for optimal absorbance of menses and other fluids. Also, by having a stronger, smoother outer layer of second web material 15, the interior first web material 11 is not exposed to the forces and frictions of the machinery it is run through and fibers of the interior material cannot detach themselves. If too many fibers would detach during the production process, not only would this cause deterioration in the produced tampons, but this could also clog up the machinery that produces the tampons. Lastly and most importantly, it is of the highest importance for the tampon that as few as possible absorbent fibers can detach themselves during use, as these could otherwise accumulate in the body of the consumer. By having a stronger, outer layer of second web material 15 as can be seen in FIG. 8, the fibers of the interior first web material 11 will not detach as easily as they are less exposed to forces or frictions, and even should they detach themselves, will likely be contained within the outer layer of second web material 15.

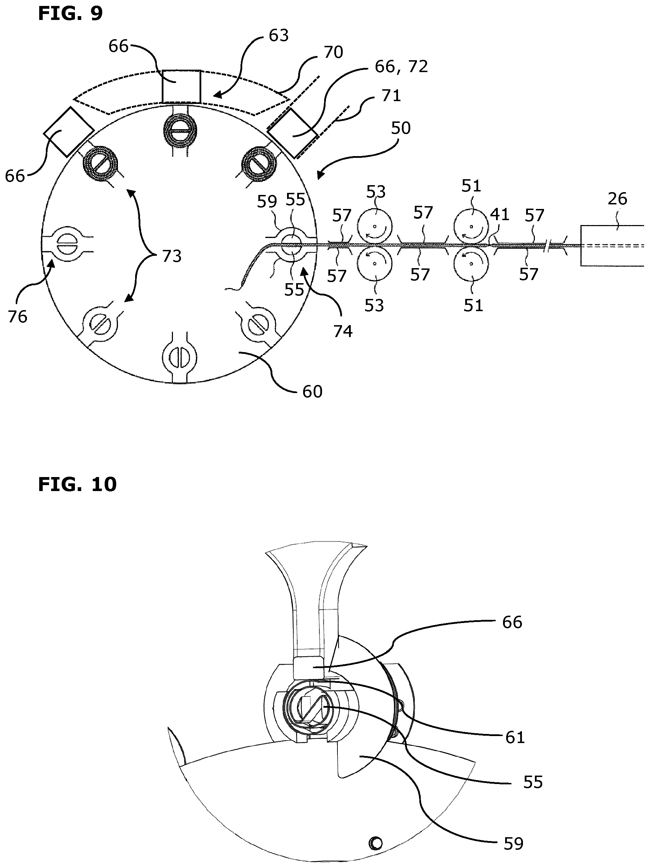

After the bonding station 26, the partially laminated structure 45 is conveyed towards a winding station 50 that will be described hereafter, by a plurality of pulling rollers 51,53 as illustrated in FIG. 9. The apparatus as illustrated in FIG. 9 comprises a first pair of rollers 51, mounted on a first frame with a distance in between the first pair of rollers 51 that is smaller than the thickness of the partially laminated structure 45. The apparatus can further comprise a second pair of rollers 53 mounted on the first or a second frame with a distance in between the second pair of rollers 53 that is smaller than the thickness of the partially laminated structure 45. The distances in between said pairs of rollers 51, 53 are thereby suitable for pulling the first and second web material 11,15.

The first pair of rollers 51 is configured to rotate and thereby frictionally pull the endless laminated structure 45 with a first linear speed. The second pair of rollers 53 is configured to rotate and thereby pull the endless absorbent fiber 1 sheet with essentially the first linear speed. The rolls can thereby be driven by at least one motor.

During in-line processing, the partially bonded first and second web material 11,15 are propagated from the first pair of rollers 51 to the second pair of rollers 53. From the second pair of rollers 53, the partially laminated structured 45 is propagated to a pulling means. Preferably, the pulling means comprises a rotatable clamping means 55 for clamping the partially bonded first and second web material 11,15 sheet and for tearing off, namely at the breaking point 41, and rolling up the partially laminated structure 45. The tearing off and rolling up of the partially laminated structure 45 are thereby performed by the same rotational motion of the rotatable clamping means 55.

The apparatus can comprise guiding means 57 to guide the partially bonded first and second web material 11,15 to the first pair of rollers 51 and/or from the first pair of rollers 51 to the second pair of rollers 53 and/or from the second pair of rollers 53 to the rotatable clamping means 55. Preferably the guiding means 57 comprise one or more pairs of guiding blades 57, each pair of blades 57 comprising a slit to guide the absorbent fiber sheet 1 in between the blades. The slit of each pair of guiding blades 57 is thereby thin enough for limiting substantial movement of the first and second web material 11,15.

In a preferred embodiment, the rotatable clamping means comprises a cylindrical casing 59, the cylindrical casing 59 comprising two slits to let through the teared up strip of partially bonded first and second web material 11,15. The cylindrical casing 59 is suitable for spatially restricting a rolled-up tampon preform to prevent it from unrolling during and/or after the rolling up step.

The pulling force causes bursting along the breaking points 41 and the detached strip of partially bonded first and second web material 11,15 is spirally rolled up, or wound, to a tampon preform by the rotation of the rotatable clamping means 55.

Once a spirally rolled-up tampon preform is formed on the rotatable clamping means 55, either the tampon preform should be removed from the clamping means 55 or another rotatable clamping means 55 should be provided, before the new tip of the propagated endless partially bonded first and second web material 11,15 arrives. FIG. 9 schematically represents a preferred embodiment of the system of the present invention. The apparatus comprises a transferring device, in this embodiment a wheel 60 for conveying an at least partially rolled-up strip away from the endless web material sheet, the wheel 60 comprising a wheel axis in essence parallel to the transversal direction about which the wheel 60 can be rotated. The wheel in FIG. 9 comprises eight rotatable clamping means 55. Each rotatable clamping means 55 comprises a cylindrical casing 59 for limiting the expansion of a spirally rolled-up detached strip of partially bonded first and second web material 11,15 sheet. The specific embodiment in FIG. 9 should not be interpreted as limiting. The wheel 60 can comprise any number of rotatable clamping means 55, that number can be larger than or equal to two rotatable clamping means 55, preferably at least four rotatable clamping means, more preferably at least six rotatable clamping means 55. For example, the wheel 60 can comprise eight, ten, twelve, fourteen, sixteen, eighteen or twenty rotatable clamping means 55. The wheel 60 can comprise more than twenty rotatable clamping means 55.

When a first strip of partially bonded first and second web material 11,15 arrives at a first rotatable clamping means 55, it passes through the slit of the first rotatable clamping means 55. This slit is adjusted to rotatable clamping means 55 said sheet when the breaking point 41 enters the region in between the first pair of rollers 51 and the second pair of rollers 53. After clamping, preferably shortly or immediately after clamping, the first rotatable clamping means 55 starts to rotate. The rotating clamping means 55 induces via the second pair of rolls which rotate in a freewheel-type manner a pulling force over the breaking point 41, which causes the breaking point 41 to burst and the detached strip of the partially bonded first and second web material 11,15 sheet can then be rolled up to a spirally wound tampon preform. The wheel 60 is rotated, in FIG. 9 counterclockwise from the viewing perspective in FIG. 9, but in another embodiment the rotation can be clockwise. A second rotatable clamping means 55 is thereby positioned, with its slit accordingly adjusted, to receive the new tip of partially bonded first and second web material 11,15 sheet, and the process is repeated. The spirally rolled-up tampon preform in and around the first rotatable clamping means 55 is then rotated by the wheel 60 to a sealing station 63 for sealing the second-end 61, or free-end 61, (illustrated FIG. 7 and 10) of the second web material 15 that has not been bonded to the first web material 11 at the bonding station 26.

According to the invention, the sealing station 63 comprises an ultrasonic welding device. The ultrasonic welding device comprises an ultrasonic vibration generator (not illustrated) and a sealing sonotrode 66. The sealing station 63 is positioned at proximity of the wheel 60. For example, the sealing station 63 can be arranged on the vertical top portion of the wheel 60 as illustrated in FIG. 9. In other words, when the sealing station 63 is arranged on the vertical top position, or portion, of the wheel 60, the axis passing through the sealing sonotrode 66 and the center of the wheel 60 is perpendicular to the axis passing through the pick-up location 74 (described hereunder) and the center of the wheel 60. The rotatable clamping means 55 are calibrated in such a way that when the rolled-up, or wound, tampon preform comes up to the sealing station 63, the free-end 61 is positioned on top so that the sealing sonotrode 66 can transmit the vibrational energy and selectively melt the second web material 15 in a process similar to the bonding station 26 as described above. The tampon preform is thereby sealed up with the outer layer corresponding to the second web material 15 around the absorbent material, or first web material 11 with a seal 67, as can be seen in FIG. 11.

In order to ensure that the tampon is correctly sealed, according to an embodiment, the sealing station 63 can comprise more than one sealing sonotrode 66. According to different embodiments, the sealing station 63 can comprise one, two, three, four, five or more sealing sonotrodes 66. This way, should the tampon be rotated too much or not enough resulting in the free-end 61 being offset from the sealing sonotrode 66 at the top position, the sealing sonotrodes 66 placed at different positions of the wheel 60 can melt the free-end 61 of the second web material thus properly sealing the tampon.

According to an embodiment, the sealing station 63 can comprise a deflecting plate near the sealing sonotrode 66 that is arranged in a manner as to ensure that the free-end 61 of the wound tampon stays within the cylindrical casing 59 and that said free-end 61 is properly guided to the sealing sonotrode 66 and its welding surface.

According to another embodiment, the sealing sonotrode 66 can be mounted mobile relative to the wheel 60. The sealing sonotrode 66 can be arranged within a railing 70 system and follow a defined track 70, or cam track, as illustrated in FIG. 9. In order to be constantly in proximity to the wheel 60 and the rotatable clamping means 55, the railing or track 70 is of complementary shape of the wheel 60, i.e. the railing or track is curved, or arcuate. The sealing sonotrode 66 can move along the wheel 60 at the same speed of the wheel 60 in order to ensure that the tampon preform, which rotates thanks to the rotatable clamping means 55, will inevitably pass through a position where the free-end 61 is in contact, or close to, the sealing sonotrode 66. In this embodiment, the sealing sonotrode 66 is actuated and moves at an constant distance relative to the rotatable clamping means 55. In other words, the tampon will be sealed since the free-end 61 is inevitably melted. The railing 70 can span over a degree comprised between 0° and 180°, preferably between 30° and 120°, more preferably between 45° and 90°, in other words the angle defined between the first end of the railing 70 and the opposite second end of the railing is comprised between these values, for example the railing 70 corresponds to an arc that extends up to 60°.

According to another embodiment, the sealing station 63 can comprise two stationary sealing sonotrodes 66 and one mobile sealing sonotrode 66 arranged in between. The sealing station 63 can also comprise two mobile sealing sonotrodes 66, for example moving in mirror or opposite directions, i.e. if one is moving clockwise then the other is moving counter-clockwise and vice versa.

According to another embodiment, the sealing station 63, more specifically the sealing sonotrode 66 can comprise an elevating mechanism to enable the movement of the sealing sonotrode 66 on one axis. In other words, the sealing station 63 comprises a actuator and an at least partially linear rail 71, or sliding guide 71, to move the sealing sonotrode 66 up and down, or in this case radially closer or remoter to the wheel 60, clamping means 55 and the clamped tampon blank. The elevating mechanism is preferably a reciprocating mechanism inducing a back and forth linear motion. Such elevating mechanisms can comprise for example and not limited to, scotch yoke, slider-crank, piston engine, traction elevator geared or gearless, turbine, spring. More specifically, the sealing sonotrode 66 can be fixed to a frame with said frame being actuated or moved back and forth in a linear movement by an elevating mechanism as described hereabove. This ensures that the sealing sonotrode 66 is in close contact with the free-end 61 of the tampon thereby improving the sealing step efficiency.

As illustrated in FIG.9, the wheel 60 comprises a pick-up location 74 corresponding to the point when the first strip of partially bonded first and second web material 11,15 arrives at a first rotatable clamping means 55 and passes through the slit of the first rotatable clamping means 55. The wheel 60 also comprises a drop-off location 76 corresponding to the point where the tampon once sealed-up can then be picked-up by an arm or pushed out of the rotatable clamping means 55 and transferred to a compressive device to give the desired shaped to the tampon.

The ultrasonic welding takes up a few milliseconds, for example from 1 to 20 milliseconds. Thus, the tampon is sequentially, or intermittently, transferred from the pick-up location 74 to the drop-off location 76. In other words, the tampon blank, sealed or unsealed, passes through intermediate stations 73 where the wheel 60 pauses for a few milliseconds and then rotates again. During these pauses, the sealing sonotrode 66 induces vibrations to the free-end 61 to melt the second web material 15 and seal-up the tampon. The picking or pushing of the sealed tampon at the drop-off location 76 is configured to take the same amount of time, i.e. a few milliseconds, or less time if the at least one sealing sonotrode 66 is mounted mobile in rotation along an arcuate railing 70, given that the ultrasonic welding can be continuous. The same can be said for the amount of time to wind the tampon at the pick-up location 74.

As illustrated in FIG. 9, the wheel 60 comprises eight intermediate stations 73, in which when the rotatable clamping means 55 all simultaneously reach an intermediate station 73, the wheel 60 stops, or pauses, for a few milliseconds. Of course, the wheel 60 can comprise more rotatable clamping means 55 and intermediate stations 73.

According to another embodiment, the sealing station 63 comprises a single sealing sonotrode 66 that is arranged at a position 72 which is shifted, or at an angle, from the top position as illustrated in FIG. 9. As illustrated in FIG.9, the sealing sonotrode 66 is preferably positioned at an intermediate station 73 that is located after the pick-up location 74 and prior to the location arranged at the top of the wheel 60. For example, the sealing sonotrode 66 can be arranged at a position of the wheel 60 which defines an angle of 45° with the axis passing through the center of the wheel 60 and the pick-up location 74 as illustrated in FIG. 9. Of course the sealing sonotrodre 66 can be arranged at a position of the wheel 60 defining said angle comprised between 5° and 85°, preferably between 20° and 70°.

The sealing sonotrode 66 is arranged at an intermediate station 73 where the rotatable clamping means 55 and the free-end 61 are aligned so that one of the rotatable clamping means 55 can act as an anvil for the sonotrode thereby improving the sealing.

## Claims

1. Method for manufacturing a tampon, comprising the steps of:

   a. supplying a continuous first web material (11);
   b. supplying a continuous second web material (15);
   c. cutting strips of the continuous second web material (15), whereby the strips are cut transversally, preferably perpendicularly, with respect to a longitudinal axis of the continuous second web material (15) and equidistantly repeated over the longitudinal axis of the continuous second web material (15);
   d. bonding a first-end (43) of the strip of second web material (15) to the continuous first web material (11), thereby forming a partially laminated structure (45) at a bonding station (26);
   e. conveying said partially laminated structure (45) to a winding station (50);
   f. clamping the partially laminated structure (45) by rotatable clamping means (55) and rolling-up the clamped partially laminated structure (45), thereby separating the partially laminated structure (45) into strips along breaking points;
   g. winding the partially laminated structure (45) strip so that the strip of second web material (15) forms the outer layer around the first web material (11);
   h. transferring the wound partially laminated structure (45) strip to a sealing station (63); and
   i. sealing the second-end (61) of the strip of second web material (15) to the first web material (11) and/or second web material (15),

**characterized in that** the sealing step comprises ultrasonic bonding, wherein the bonding step concurrently comprises an embossing step where the bonding station (28) comprise an embossment roller (28) that has spaced apart protrusions (34), the partially laminated structure being arranged in a way that the first web material (11) comes into contact with the embossment roller (28).

2. Method according to the preceding claim 1, **characterized in that** the sealing station (63) comprises at least one sealing sonotrode (66) inducing vibrations at a frequency between 10 kHz and 50 kHz, preferably between 20 kHz and 35 kHz.

3. Method according to the preceding claim 2, **characterized in that** the sealing station (63) comprises three or five sealing sonotrodes (66) inducing vibrations at a frequency between 10 kHz and 50 kHz, preferably between 20 kHz and 35 kHz.

4. Method according to the preceding claim 2 or 3, **characterized in that** the at least one sealing sonotrode (66) is mounted mobile within an arcuate railing (70).

5. Method according to the preceding claims 2 or 3, **characterized in that** the at least one sealing sonotrode (66) is mounted mobile within an at least partially linear rail (71).

6. Method according to at least one of the preceding claims 1 to 5, wherein the method further comprises a step of applying predetermined breaking points (41) on the continuous first web material (11), whereby the predetermined breaking points (41) are applied transversally with respect to a longitudinal axis of the continuous first web material (11) and equidistantly repeated over the longitudinal axis of the continuous first web material (11), and whereby said step of applying predetermined breaking points (41) is executed prior to the bonding step.

7. Apparatus for manufacturing a tampon, comprising:

   - a first conveying system comprising:

      i. one or more conveying means, preferably comprising one or more rollers (12,14,24), for conveying a first web material (11) to a bonding station (26);

   - a second conveying system comprising:

      i. one or more conveying means, preferably comprising one or more rollers (16,18,20,22,24), for conveying the second web material (15) to the bonding station (26);

      ii. a separation station (20,22), preferably comprising a cutting roller (20) and a counterpressure roller (22), for cutting strips of second web material (15), preferably configured for cutting said strips equidistantly and transversally, more preferably perpendicularly, with respect to a conveying direction of the second web material (15);

   - a bonding station (26), for bonding a first-end (43) of the strip of second web material (15) to the first web material (11); thereby forming a partially laminated structure (45);
   - a winding station (50) comprising rotatable clamping means (55), for clamping and winding the partially laminated structure (45);
   - a sealing station (63), for sealing the second-end (61) of the strip of second web material (15) to the first web material (11) and/or second web material (15);

   **characterized in that** the sealing station (63) comprises an ultrasonic welding device (66) inducing vibrations, wherein the bonding station (26) comprises an embossment roller (28) comprising a smooth surface (31) and a embossed surface (29), the embossed surface (29) comprising an embossment pattern (29a) with at least one row ($29r_m$) of protrusions (34), said protrusions being arranged in the same orientation for each row and being spaced apart from one another.

8. Apparatus for manufacturing a tampon according to the claim 7, wherein the ultrasonic welding device is at least one sealing sonotrode (66) inducing vibrations at a frequency between 10 kHz and 50 kHz, preferably between 20 kHz and 35 kHz.

9. Apparatus according to the preceding claim 7 or 8, wherein the apparatus further comprises a weakening station (12,14), said weakening station preferably comprising a weakening roller (14), more preferably a perforation roller, and a counterpressure roller (12), for applying predetermined breaking points (41) on the first web material (11) or on the partially laminated structure (45), whereby the predetermined breaking points (41) are applied transversally with respect to a longitudinal axis of the continuous first web material (11) and equidistantly repeated over the longitudinal axis of the continuous first web material (11).

10. Apparatus according to any of the preceding claims 7 to 9, **characterized in that** the sealing station (63) comprises a reciprocating mechanism enabling the movement of the at least one sealing sonotrode (66) closer or remoter to the rotatable clamping means

**11.** Apparatus according to any of the preceding claim 7 to 10, wherein the bonding station (26) comprises an ultrasonic welding device to bond the first-end (43) of the second web material (15) to the first web material (11), the ultrasonic welding device comprising a bonding sonotrode (32) inducing vibrations at a frequency between 10 kHz and 50 kHz, preferably between 20 kHz and 35 kHz.

**Patentansprüche**

**1.** Verfahren zum Herstellen eines Tampons, umfassend die Schritte:

a. Zuführen eines kontinuierlichen ersten Bahnmaterials (11);
b. Zuführen eines kontinuierlichen zweiten Bahnmaterials (15);
c. Schneiden von Streifen aus dem kontinuierlichen zweiten Bahnmaterial (15), wodurch die Streifen quer, vorzugsweise senkrecht, hinsichtlich einer Längsachse des kontinuierlichen zweiten Bahnmaterials (15) geschnitten werden und sich über die Längsachse des kontinuierlichen zweiten Bahnmaterials (15) äquidistant wiederholen;
d. Bonden eines ersten Endes (43) des Streifens aus zweitem Bahnmaterial (15) mit dem kontinuierlichen ersten Bahnmaterial (11), wobei dadurch an einer Bonding-Station (26) eine teilweise laminierte Struktur (45) ausgebildet wird;
e. Befördern der teilweise laminierten Struktur (45) zu einer Wickelstation (50);
f. Einklemmen der teilweise laminierten Struktur (45) durch drehbare Klemmmittel (55) und Aufrollen der eingeklemmten teilweise laminierten Struktur (45), wobei dadurch die teilweise laminierte Struktur (45) entlang von Sollbruchstellen in Streifen getrennt wird;
g. Aufwickeln des Streifens der teilweise laminierten Struktur (45), so dass der Streifen aus zweitem Bahnmaterial (15) die äußere Schicht um das erste Bahnmaterial (11) herum ausbildet;
h. Übertragen des aufgewickelten Streifens der teilweise laminierten Struktur (45) zu einer Versiegelungsstation (63); und
i. Versiegeln des zweiten Endes (61) des Streifens aus zweitem Bahnmaterial (15) mit dem ersten Bahnmaterial (11) und/oder dem zweiten Bahnmaterial (15),

**dadurch gekennzeichnet, dass** der Versiegelungsschritt ein Ultraschallbonden umfasst, wobei der Bonding-Schritt gleichzeitig einen Prägeschritt umfasst, bei dem die Bonding-Station (28) eine Prägewalze (28) umfasst, die beabstandete Vorsprünge (34) aufweist, wobei die teilweise laminierte Struktur auf eine Weise angeordnet ist, dass das erste Bahnmaterial (11) mit der Prägewalze (28) in Kontakt kommt.

**2.** Verfahren nach dem vorstehenden Anspruch 1, **dadurch gekennzeichnet, dass** die Versiegelungsstation (63) mindestens eine Versiegelungssonotrode (66) umfasst, die Schwingungen bei einer Frequenz zwischen 10 kHz und 50 kHz, vorzugsweise zwischen 20 kHz und 35 kHz, induziert.

**3.** Verfahren nach dem vorstehenden Anspruch 2, **dadurch gekennzeichnet, dass** die Versiegelungsstation (63) drei oder fünf Versiegelungssonotroden (66) umfasst, die Vibrationen bei einer Frequenz zwischen 10 kHz und 50 kHz, vorzugsweise zwischen 20 kHz und 35 kHz, induzieren.

**4.** Verfahren nach dem vorstehenden Anspruch 2 oder 3, **dadurch gekennzeichnet, dass** die mindestens eine Versiegelungssonotrode (66) innerhalb eines bogenförmigen Geländers (70) bewegbar montiert ist.

**5.** Verfahren nach den vorstehenden Ansprüchen 2 oder 3, **dadurch gekennzeichnet, dass** die mindestens eine Versiegelungssonotrode (66) innerhalb einer mindestens teilweise geradlinigen Schiene (71) bewegbar montiert ist.

**6.** Verfahren nach mindestens einem der vorstehenden Ansprüche 1 bis 5, wobei das Verfahren ferner einen Schritt eines Anbringens von zuvor bestimmten Sollbruchstellen (41) auf dem kontinuierlichen ersten Bahnmaterial (11) umfasst, wodurch die zuvor bestimmten Sollbruchstellen (41) quer hinsichtlich einer Längsachse des kontinuierlichen ersten Bahnmaterials (11) angebracht und über die Längsachse des kontinuierlichen ersten Bahnmaterials (11) äquidistant wiederholt werden, und wodurch der Schritt des Anbringens von zuvor bestimmten Sollbruchstellen (41) vor dem Bonding-Schritt ausgeführt wird.

**7.** Einrichtung zum Herstellen eines Tampons, umfassend:

- ein erstes Fördersystem, umfassend:

i. ein oder mehrere Fördermittel, vorzugsweise umfassend eine oder mehrere Walzen (12,14,24) zum Fördern eines ersten Bahnmaterials (11) zu einer Bonding-Station (26);

- ein zweites Fördersystem, umfassend:

i. ein oder mehrere Fördermittel, vorzugsweise umfassend eine oder mehrere Walzen (16,18,20,22,24), zum Fördern des zweiten Bahnmaterials (15) zu der Bonding-Station (26);

ii. eine Trennungsstation (20,22), vorzugsweise umfassend eine Schneidwalze (20) und eine Gegendruckwalze (22), zum Schneiden von Streifen aus zweitem Bahnmaterial (15), vorzugsweise konfiguriert zum Schneiden der Streifen äquidistant und quer, mehr bevorzugt senkrecht, hinsichtlich eine Förderrichtung des zweiten Bahnmaterials (15);

- eine Bonding-Station (26) zum Bonden eines ersten Endes (43) des Streifens aus zweitem Bahnmaterial (15) mit dem ersten Bahnmaterial (11);

wobei dadurch eine teilweise laminierte Struktur (45) ausgebildet wird;

- eine Wickelstation (50), umfassend drehbare Klemmmittel (55) zum Klemmen und Wickeln der teilweise laminierten Struktur (45);

- eine Versiegelungsstation (63) zum Versiegeln des zweiten Endes (61) des Streifens aus zweitem Bahnmaterial (15) mit dem ersten Bahnmaterial (11) und/oder dem zweiten Bahnmaterial (15);

**dadurch gekennzeichnet, dass** die Versiegelungsstation (63) eine Ultraschallschweißvorrichtung (66) umfasst, die Vibrationen induziert, wobei die Bonding-Station (26) eine Prägewalze (28) umfasst, umfassend eine glatte Oberfläche (31) und eine geprägte Oberfläche (29), die geprägte Oberfläche (29) umfassend ein Prägemuster (29a) mit mindestens einer Reihe (29$r_m$) von Vorsprüngen (34), wobei die Vorsprünge für jede Reihe in der gleichen Ausrichtung angeordnet und voneinander beabstandet sind.

8. Einrichtung zum Herstellen eines Tampons nach Anspruch 7, wobei die Ultraschallschweißvorrichtung mindestens eine Versiegelungssonotrode (66) ist, die Vibrationen bei einer Frequenz zwischen 10 kHz und 50 kHz, vorzugsweise zwischen 20 kHz und 35 kHz, induziert.

9. Einrichtung nach dem vorstehenden Anspruch 7 oder 8, wobei die Einrichtung ferner eine Schwächungsstation (12,14) umfasst, die Schwächungsstation vorzugsweise umfassend eine Schwächungswalze (14), mehr bevorzugt eine Perforationswalze, und eine Gegendruckwalze (12) zum Anbringen von zuvor bestimmten Sollbruchstellen (41) auf dem ersten Bahnmaterial (11) oder auf der teilweise laminierten Struktur (45), wodurch die zuvor bestimmten Sollbruchstellen (41) quer hinsichtlich einer Längsachse des kontinuierlichen ersten Bahnmaterials (11) angebracht sind und sich über die Längsachse des kontinuierlichen ersten Bahnmaterials (11) äquidistant wiederholen.

10. Einrichtung nach einem der vorstehenden Ansprüche 7 bis 9,

**dadurch gekennzeichnet, dass** die Versiegelungsstation (63) einen Hin- und Herbewegungsmechanismus umfasst, der die Bewegung der mindestens einen Versiegelungssonotrode (66) näher an die drehbaren Klemmmittel (55) oder davon weg ermöglicht.

11. Einrichtung nach einem der vorstehenden Ansprüche 7 bis 10, wobei die Bonding-Station (26) eine Ultraschallschweißvorrichtung umfasst, um das erste Ende (43) des zweiten Bahnmaterials (15) mit dem ersten Bahnmaterial (11) zu bonden, die Ultraschallschweißvorrichtung umfassend eine Bonding-Sonotrode (32), die Vibrationen bei einer Frequenz zwischen 10 kHz und 50 kHz, vorzugsweise zwischen 20 kHz und 35 kHz, induziert.

**Revendications**

1. Procédé de fabrication d'un tampon, comprenant les étapes consistant à :

a. fournir un premier matériau de voile (11) continu ;

b. fournir un second matériau de voile (15) continu ;

c. découper des bandes du second matériau de voile (15) continu, moyennant quoi les bandes sont découpées transversalement, de préférence perpendiculairement, par rapport à un axe longitudinal du second matériau de voile (15) continu et répétées de manière équidistante sur l'axe longitudinal du second matériau de voile (15) continu ;

d. coller une première extrémité (43) de la bande de second matériau de voile (15) au premier matériau de voile (11) continu, formant de ce fait une structure partiellement stratifiée (45) au niveau d'un poste de collage (26) ;

e. transporter ladite structure partiellement stratifiée (45) vers un poste de bobinage (50) ;

f. serrer la structure partiellement stratifiée (45) par des moyens de serrage rotatifs (55) et enrouler la structure partiellement stratifiée (45) serrée, séparant de ce fait la structure partiellement stratifiée (45) en bandes le long de points de rupture ;

g. bobiner la bande de structure partiellement stratifiée (45) de sorte que la bande de second matériau de voile (15) forme la couche externe autour du premier matériau de voile (11) ;

h. transférer la bande de structure partiellement stratifiée (45) bobinée vers un poste de scellage (63) ; et

i. sceller la seconde extrémité (61) de la bande de second matériau de voile (15) au premier matériau de voile (11) et/ou au second matériau de voile (15),

**caractérisé en ce que** l'étape de scellage comprend un collage par ultrasons, dans lequel l'étape de collage comprend simultanément une étape de gaufrage où le poste de collage (28) comprend un rouleau de gaufrage (28) qui a des protubérances (34) espacées, la structure partiellement laminée étant agencée de manière à ce que le premier matériau de voile (11) entre en contact avec le rouleau de gaufrage (28).

2. Procédé selon la revendication précédente 1, **caractérisé en ce que** le poste de scellage (63) comprend au moins une sonotrode de scellage (66) induisant des vibrations à une fréquence comprise entre 10 kHz et 50 kHz, de préférence entre 20 kHz et 35 kHz.

3. Procédé selon la revendication précédente 2, **caractérisé en ce que** le poste de scellage (63) comprend trois ou cinq sonotrodes de scellage (66) induisant des vibrations à une fréquence comprise entre 10 kHz et 50 kHz, de préférence entre 20 kHz et 35 kHz.

4. Procédé selon la revendication précédente 2 ou 3, **caractérisé en ce que** l'au moins une sonotrode de scellage (66) est montée mobile au sein de rails arqués (70).

5. Procédé selon les revendications précédentes 2 ou 3,
**caractérisé en ce que** l'au moins une sonotrode de scellage (66) est montée mobile au sein d'un rail (71) au moins partiellement linéaire.

6. Procédé selon au moins l'une des revendications précédentes 1 à 5, dans lequel le procédé comprend en outre une étape consistant à appliquer des points de rupture (41) prédéterminés sur le premier matériau de voile (11) continu, moyennant quoi les points de rupture (41) prédéterminés sont appliqués transversalement par rapport à un axe longitudinal du premier matériau de voile (11) continu et répétés de manière équidistante sur l'axe longitudinal du premier matériau de voile (11) continu, et moyennant quoi ladite étape consistant à appliquer des points de rupture (41) prédéterminés est exécutée avant l'étape de collage.

7. Appareil de fabrication d'un tampon, comprenant :

- un premier système de transport comprenant :

i. un ou plusieurs moyens de transport, comprenant de préférence un ou plusieurs rouleaux (12, 14, 24), pour transporter un premier matériau de voile (11) vers un poste de collage (26) ;

- un second système de transport comprenant :

i. un ou plusieurs moyens de transport, comprenant de préférence un ou plusieurs rouleaux (16, 18, 20, 22, 24), pour transporter le second matériau de voile (15) vers le poste de collage (26) ;
ii. un poste de séparation (20, 22), comprenant de préférence un rouleau de découpe (20) et un rouleau de contre-pression (22), pour découper des bandes du second matériau de voile (15), de préférence conçu pour découper lesdites bandes à équidistance et transversalement, plus préférablement perpendiculairement, par rapport à une direction de transport du second matériau de voile (15) ;

- un poste de collage (26), pour coller une première extrémité (43) de la bande de second matériau de voile (15) au premier matériau de voile (11) ; formant de ce fait une structure partiellement stratifiée (45) ;
- un poste de bobinage (50) comprenant des moyens de serrage rotatifs (55), pour serrer et bobiner la structure partiellement stratifiée (45) ;
- un poste de scellage (63), pour sceller la seconde extrémité (61) de la bande de second matériau de voile (15) au premier matériau de voile (11) et/ou au second matériau de voile (15) ;

**caractérisé en ce que** le poste de scellage (63) comprend un dispositif de soudage par ultrasons (66) induisant des vibrations, dans lequel le poste de collage (26) comprend un rouleau de gaufrage (28) comprenant une surface lisse (31) et une surface gaufrée (29), la surface gaufrée (29) comprenant un motif de gaufrage (29a) avec au moins une rangée (29$r_m$) de saillies (34), lesdites saillies étant agencées dans la même orientation pour chaque rangée et étant espacées l'une de l'autre.

8. Appareil de fabrication d'un tampon selon la revendication 7, dans lequel le dispositif de soudage par ultrasons est au moins une sonotrode de scellage (66) induisant des vibrations à une fréquence comprise entre 10 kHz et 50 kHz, de préférence entre 20 kHz et 35 kHz.

9. Appareil selon la revendication précédente 7 ou 8, dans lequel l'appareil comprend en outre un poste d'affaiblissement (12, 14), ledit poste d'affaiblissement comprenant de préférence un rouleau d'affaiblissement (14), plus préférablement un rouleau de perforation, et un rouleau de contre-pression (12), pour appliquer des points de rupture (41) prédéterminés sur le premier matériau de voile (11) ou sur la structure partiellement stratifiée (45), moyennant quoi les points de rupture (41) prédéterminés sont appliqués transversalement par rapport à un axe longitudinal du premier matériau de voile (11) continu et répétés de manière équidistante sur l'axe longitudinal du premier matériau de voile (11) continu.

10. Appareil selon l'une quelconque des revendications précédentes 7 à 9, **caractérisé en ce que** le poste de scellage (63) comprend un mécanisme alternatif permettant le mouvement de l'au moins une sonotrode de scellage (66) en rapprochement ou en éloignement des moyens de serrage rotatifs (55).

11. Appareil selon l'une quelconque des revendications précédentes 7 à 10, dans lequel le poste de collage (26) comprend un dispositif de soudage par ultrasons pour coller la première extrémité (43) du second matériau de voile (15) au premier matériau de voile (11), le dispositif de soudage par ultrasons comprenant une sonotrode de collage (32) induisant des vibrations à une fréquence comprise entre 10 kHz et 50 kHz, de préférence entre 20 kHz et 35 kHz.

**FIG. 1**

**FIG. 2**

**FIG. 3**

**Fig. 4**

**FIG. 5**

29r5
29r4
29r3
29r2
29r1
28

**FIG. 6**

35
32
26
34
37
15
11
28
31
29
39

**FIG. 7**

**FIG. 8**

**FIG. 9**

**FIG. 10**

FIG. 11

**EP 4 014 935 B1**

**Patent documents cited in the description**

- EP 1189566 A
- EP 3165471 A
- EP 3184092 A